# EUROPÄISCHE PATENTANMELDUNG

(11) **EP 0 648 511 A2**
(43) Veröffentlichungstag der Anmeldung: **19.04.1995**
(21) Anmeldenummer: 94116335.4
(22) Anmeldetag: 17.10.1994
(51) Int. Cl.: A61M 5/34, A61M 5/32, A61M 5/50

(54) **Injektionsspritze**

(30) Priorität: 18.10.1993 DE 9315861 U
(71) Anmelder: MEDI plus TEC Medizinisch-technische Handelsgesellschaft mbH, D-47441 Moers (DE)
(72) Erfinder: Wirtz, Matthias, D 47445 Moers (DE)
(74) Vertreter: Weickmann, Heinrich, Dipl.-Ing.

(57) **Zusammenfassung**

Die Erfindung betrifft eine Injektionsspritze 1 mit einem Zylinder 2, an dessen einem Ende ein eine Injektionsnadel 11 halternder Nadelhalter 4 anbringbar ist, mit einem in dem Zylinder 2 verschiebbaren Kolben 6, an dem eine aus dem anderen Ende des Zylinders 2 herausragende, an ihrem Ende mit einem Betätigungsgriff 23 versehene Kolbenstange 5 angebracht ist, und mit einer Vorrichtung zur selbsttätigen Verriegelung des über ein vorgegebenes Maß in Richtung zur Injektionsnadel 11 vorgeschobenen Kolbens 6 an dem Nadelhalter 4 gegenüber Zurückziehen des Kolbens 6, bei der der Nadelhalter 4 durch eine durch Hineindrehen und gegebenenfalls axiales Verschieben des Nadelhalters 4 in den Zylinder 2 zu lösende Drehverbindung 8, 15 mit dem Zylinder 2 verbindbar ist und bei der sich an dem Nadelhalter 4 und dem Kolben 6 Drehsicherungsglieder 18, 21 befinden, die bei Vorschieben des Kolbens 6 in Richtung zum Nadelhalter 4 zur Verrigelung des Kolbens 6 an dem Nadelhalter 4 ineinandergreifen und durch Verdrehen des Betätigungsglieds 23 eine Lösung der Drehverbindung 8, 15 und daraufhin ein Hineinziehen des Kolbens 6, des Nadelhalters 4 und der Injektionsnadel 11 über ihre ganze Länge in den Zylinder 2 gestatten, wobei die Drehsicherungsglieder 18, 21 Winkelabstände zueinander aufweisende, durch axiales Vorschieben des Kolbens 6 in gegenseitigen, kämmenden Eingriff zu bringende Stege 18, 21 sind, dadurch gekennzeichnet, daß zwischen dem Innenumfang des Zylinders 2 und dem Außenumfang des Nadelhalters 4 Verrastungsmittel 31, 32 vorgesehen sind, die ein verhältnismäßig leichtes Eindrehen der Drehverbindung 8, 15 zwischen dem Nadelhalter 4 und dem Zylinder 2 und ein verhältnismäßig schweres Ausdrehen der Drehverbindung 8, 15 bewirkt.

## Beschreibung

Die Erfindung betrifft eine Injektionsspritze mit einem Zylinder, an dessen einem Ende ein eine Injektionsnadel halternder Nadelhalter anbringbar ist, mit einem in dem Zylinder verschiebbaren Kolben, an dem eine aus dem anderen Ende des Zylinders herausragende, an ihrem Ende mit einem Betätigungsgriff versehene Kolbenstange angebracht ist, und mit einer Vorrichtung zur selbsttätigen Verriegelung des über ein vorgegebenes Maß in Richtung zur Injektionsnadel vorgeschobenen Kolbens an dem Nadelhalter gegenüber Zurückziehen des Kolbens, bei der der Nadelhalter durch eine durch Hineindrehen und gegebenenfalls axiales Verschieben des Nadelhalters in den Zylinder zu lösende Drehverbindung mit dem Zylinder verbindbar ist und bei der sich an dem Nadelhalter und dem Kolben Drehsicherunsglieder befinden, die bei Vorschieben des Kolbens in Richtung zum Nadelhalter zur Verriegelung des Kolbens an dem Nadelhalter ineinandergreifen und durch Verdrehen des Betätigungsglieds eine Lösung der Drehverbindung und daraufhin ein Hineinziehen des Kolbens, des Nadelhalters und der Injektionsnadel über ihre ganze Länge in den Zylinder gestatten, wobei die Drehsicherungsglieder Winkelabstände zueinander aufweisende, durch axiales Vorschieben des Kolbens in gegenseitigen, kämmenden Eingriff zu bringende Stege sind.

Eine solche Injektionsspritze ist nach der europäischen Patentanmeldung Veröffentlichungs-Nr. 402 908 bekannt. Bei dieser Injektionsspritze kann der Nadelhalter verhältnismäßig leicht aus seiner Drehverbindung mit dem Zylinder gelöst werden.

Häufig sind Medikamente in Injektionsspritzen mittels einer Nadel zu ziehen, die einen Verschluß, etwa aus Gummi oder Kunststoff, zu durchstoßen hat, bevor sie in ein das Medikament enthaltendes Gefäß eindringen kann. Zu diesem Zweck muß eine verhältnismäßig starke Nadel verwendet werden, die meist einen verhältnismäßig großen Innendurchmesser hat. Das hat zur Folge, daß Bestandteile des durchstoßenen Verschlusses, also etwa Gummi- oder Kunststoffteilchen, in die Injektionsspritze hineingezogen werden können. Solche Teilchen dürfen keinesfalls injiziert werden. Aus diesem Grunde muß die Nadel, die zum Aufziehen des Medikaments dient, durch eine Nadel mit einem kleineren Innendurchmesser vertauscht werden. Bei einem solchen Austausch ist es, wenn eine Injektionsspritze eingangs genannter Art verwendet wird, möglich, daß sich der Nadelhalter etwas löst und somit beim Injizieren Medikament zwischen dem Nadelhalter und dem Zylinder ausfließt.

Um dies zu vermeiden, ist die Injektionsspritze dadurch gekennzeichnet, daß zwischen dem Innenumfang des Zylinders und dem Außenumfang des Nadelhalters Verrastungsmittel vorgesehen sind, die ein verhältnismäßig leichtes Eindrehen der Drehverbindung zwischen dem Nadelhalter und dem Zylinder und ein verhältnismäßig schweres Ausdrehen der Drehverbindung bewirkt.

Konstruktiv ist es besonders einfach, wenn die Verrastungsmittel durch unsymmetrische Nocken und über diese unsymmetrischen Nocken elastisch nachgiebig bewegbare Nockenfolger gebildet sind. Eine herstellungstechnisch besonders einfache Verwirklichung ist dadurch gekennzeichnet, daß die Nocken die Form von Rampen und die Nockenfolger die Form von nachgiebigen Fingern haben. Dabei befinden sich bevorzugt die Nocken am Zylinder und die Nockenfolger am Nadelhalter. Eine besonders zweckmäßige Ausführungsform ist dadurch gekennzeichnet, daß sich die Nocken an der Innenfläche des Zylinders und die Nockenfolger an der zum Kolben weisenden Stirnfläche des Nadelhalters befinden. Da in diesem Fall die Nockenfolger, also insbesondere die Finger, die Möglichkeit zum Ausweichen haben müssen, ist die Injektionsspritze bevorzugt dadurch gekennzeichnet, daß sich im Umfang eines Sockels auf dem Kolben Ausnehmungen zur Aufnahme der Nockenfolger befinden.

Um sicherzugehen, daß die Nadel nicht wieder aus dem Zylinder herausgeschoben werden kann, wird häufig vorgeschrieben, daß der Kolben nach dem Zurückziehen der Nadel abgebrochen werden muß. Um dies zu erreichen, ist die Injektionsnadel bevorzugt dadurch gekennzeichnet, daß die Stege am Kolben in Richtung zum Nadelhalter durch in Umfangsrichtung aufeinander zu strebende Flanken zugespitzt sind. Diese Maßnahme hat unabhängig von den eingangs beschriebenen Maßnahmen eine selbständige erfinderische Bedeutung.

Ein besonders einfaches Mittel zum Verrasten des Kolbens an dem Nadelhalter ist dadurch gekennzeichnet, daß zwischen den radial nach außen gerichteten Begrenzungsflächen der Stege und/oder der Zwischenräume zwischen den Stegen am Kolben und den ihnen im Verriegelungszustand gegenüberstehenden, radial nach innen gerichteten Begrenzungsflächen der Zwischenräume zwischen den Stegen und/oder der Stege am Nadelhalter voneinander lösbare Rastglieder vorgesehen sind. Auch diese Maßnahme hat selbständige Bedeutung.

Im folgenden wird die Erfindung an zwei Ausführungsbeispielen unter Hinweis auf die beigefügten Zeichnungen beschrieben. Es zeigen:
- Fig. 1: einen Längsschnitt durch das Vorderteil einer Injektionsspritze gemäß einem ersten Ausführungsbeispiel;

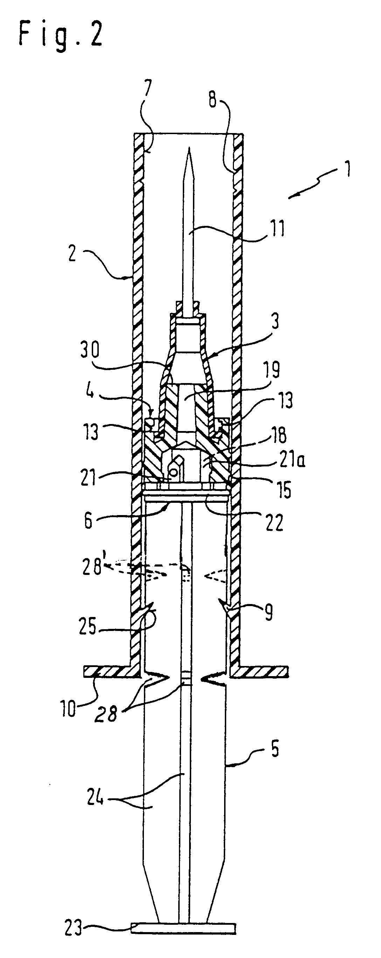

- Fig. 2: einen Längsschnitt durch die Injektionsspritze nach Fig. 1 mit zusammen mit dem Nadelhalter und der Injektionsnadel in den Zylinder zurückgezogenem, in dem Zylinder verrastetem Kolben;
- Fig. 3: einen Längsschnitt durch das Vorderteil einer Injektionsspritze gemäß einem zweiten Ausführungsbeispiel mit in dem Nadelhalter verrastetem Kolben;

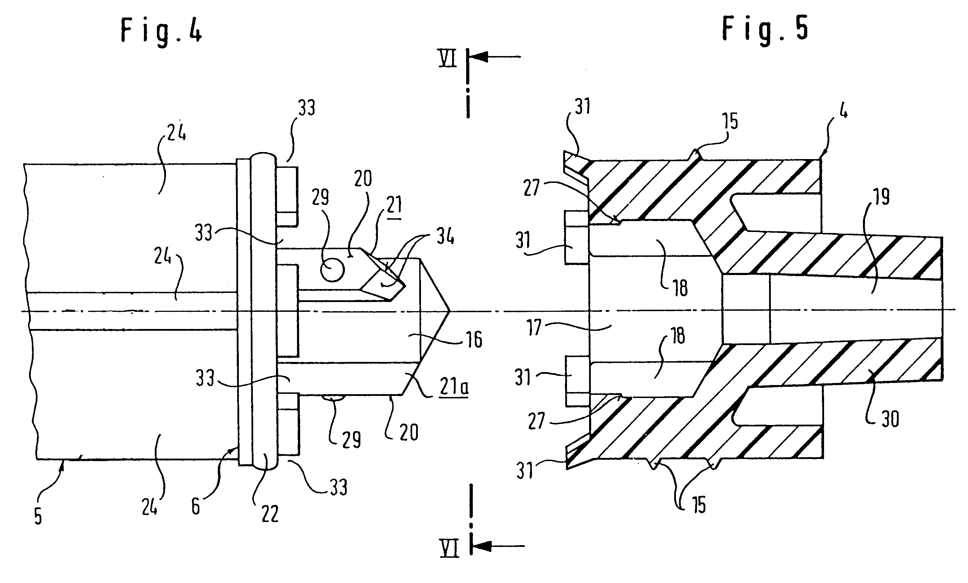

- Fig. 4: vergrößert das vordere Ende des Kolbens;
- Fig. 5: entsprechend vergrößert das hinter Ende des Nadelhalters;

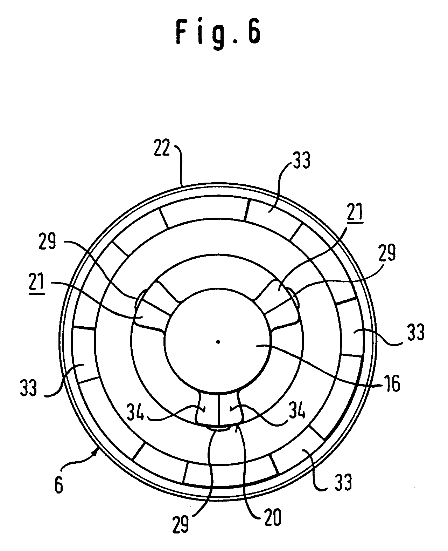

- Fig. 6: eine Ansicht entsprechend VI-VI in Fig. 4;
- Fig. 7: das Zusammenwirken der Verrastungsmittel zwischen Nadelhalter und Zylinder im Achsschnitt;
- Fig. 8: das Zusammenwirken der Verrastungsmittel zwischen Nadelhalter und Zylinder aus der Blickrichtung VIII-VIII in Fig. 7.

Die Injektionsspritze 1 weist einen Zylinder 2 konstanten Durchmessers auf, in dem eine Kolbenstange 5 verschiebbar geführt ist, eine Nadelhaltehülse 3 für eine Injektionsnadel 11, einen Nadelhalter 4 für die Nadelhaltehülse 3 und einen Kolben 6, der mit der Kolbenstange 5 einstückig ist. Der Zylinder 2 ist mit einem in seine Innenwand 7 geformten Linksgewindeabschnitt 8 versehen, in den ein Linksgewindeabschnitt 15 am Nadelhalter 4 eingeschraubt ist. In das hintere Teil des Zylinders 2 sind nasenförmige Verriegelungsvorsprünge 9 eingeformt, die in nasenförmige Verriegelungsvertiefungen 25 in im Querschnitt kreuzförmig angeordneten Führungsrippen 24 an der Kolbenstange 5 einrastbar sind. Am hinteren Ende des Zylinders 2 befinden sich außen Fingerwiderlager 10 für einen runden Betätigungsgriff 23 am freien Ende der Kolbenstange 5, mittels dem die Kolbenstange 5 verdreht und verschoben werden kann.

In dem vorderen Ende der Nadelhaltehülse 3 ist die Injektionsnadel 11 lösbar befestigt. An das hintere Ende der Nadelhaltehülse 3 ist hierzu außen ein Flansch 12 angeformt, und in eine Innenwandfläche 14 des Nadelhalters 4 ist ein Linksgewindeabschnitt 13 eingeformt, in den der Flansch 12 der Nadelhaltehülse 3 zu schrauben ist. Stattdessen kann auch eine drehgesicherte Steckverbindung zwischen der Nadelhaltehülse 3 und der Innenwandfläche 14 des Nadelhalters 4 bevorzugt sein.

Der in den Linksgewindeabschnitt 8 des Zylinders 2 geschraubte Linksgewindeabschnitt 15 ist in den Außenumfang des hinteren Mittelteils des Nadelhalters 4 eingeformt.

Der Nadelhalter 4 ist hinten mit einer Höhlung 17 versehen. Von der die Höhlung 17 hinten umgebenden Innenwand des Nadelhalters 4 stehen radial angeformte stegförmige Drehsicherungsglieder 18 hervor. Das vordere Mittelteil des Nadelhalters 4 ist von einem Kanal 19 durchsetzt, durch den zu injizierende Flüssigkeit zur Injektionsnadel 11 gelangt.

Stegförmige Drehsicherungsglieder 21 zum kämmenden Eingriff mit den Drehsicherungsgliedern 18 des Nadelhalters 4 stehen radial von einem vorderen Ansatz 16 am Kolben 6 hervor. Eines (21a) dieser Drehsicherungsglieder 21 ist in Richtung zum Nadelhalter 4 länger als die anderen. Die Drehsicherungsglieder 21 am Kolben 6 sind in Richtung zum Nadelhalter 4 durch in Umfangsrichtung aufeinander zu strebende Flanken zugespitzt. Der Umfang des Kolbens 6 ist mit einer dicht an der Innenwand 7 des Zylinders 2 anliegenden Wölbung 22 versehen. An den radial nach außen weisenden Seiten 20 der Drehsicherungsglieder 21 befinden sich Noppen 29, die zur Verriegelung des Kolbens 6 am Nadelhalter 4 hinter Innenschultern 27 zwischen den Drehsicherungsgliedern 18 im Eingang der Höhlung 17 zu drücken sind.

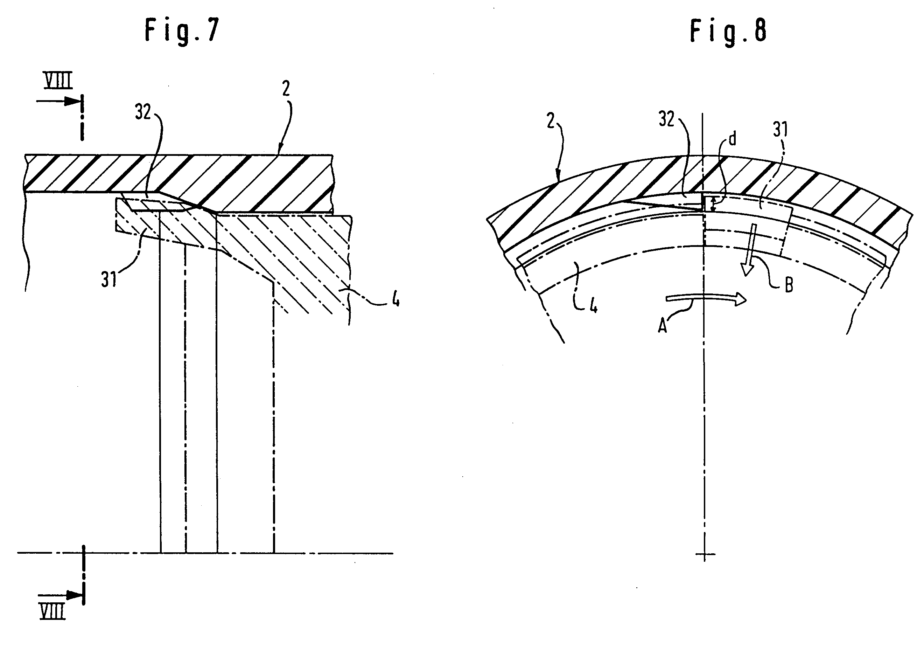

An der Innenseite des Zylinders 2 befinden sich Rampen 32 und an der zum Kolben 6 weisenden Stirnfläche des Nadelhalters 4 nachgiebige Finger 31, die beim Eindrehen des Nadelhalters 4 in den Zylinder 2 (Pfeilrichtung A in Fig. 8) über die Rampen 32 unter Ausweichen in Pfeilrichtung B (Fig. 8) gleiten und, wenn sie die Rampen 32 überschritten haben, wieder radial nach außen schnappen. Soll dann der Nadelhalter 4 wieder aus dem Zylinder 2 gedreht werden (gegensinnig zur Pfeilrichtung A), so stoßen seine Finger 31 an die Rampen 32 an und verhindern dies in beschränktem Maße. Bei einer tatsächlichen Ausführungsform ist der Abstand d in Fig. 8 nur 2/10 - 3/10 mm. Die Ausbildung kann so getroffen sein, daß die Finger 31 beim Herausdrehen des Nadelhalters 4 aus dem Zylinder 2 abgequetscht und zerstört werden, was zum Einmalgebrauch der Injektionsspritze beiträgt.

Die kreuzförmig angeordneten Führungsrippen 24 und die Kolbenstange 5 sind an dem runden Betätigungsgriff 23 festgeklebt. Die nasenförmigen Verriegelungsvertiefungen 25 im Kolben 6 verrasten nachgiebig mit den nasenförmigen Verriegelungsvorsprüngen 9 im Zylinder 2, wenn der Kolben 6 nach hinten gezogen wird, und verhindern ein vollständiges Herausziehen der Kolbenstange 5 aus dem Zylinder 2. Jedoch kann dann die Kolbenstange 5 unter Ausnutzung von in diesem Zustand aus dem Zylinder 2 herausragenden Kerben 28 in den Führungsrippen 24 leicht abgebrochen werden. Um das vollständige Herausziehen des Kolbens 6 aus dem Zylinder 2 zu verhindern, können die Verriegelungsvorsprünge 9 oder sonstige an der Innenwand 7 des Zylinders 2 angeformte Vorsprünge wie z.B. eine Umfangsrippe alternativ auch an der Rückseite des Kolbens 6 anschlagen. In diesem Fall können die Verriegelungsvertiefungen 25 entfallen. Die zum Abbrechen der Kolbenstange 5 bestimmten Kerben sind für diese Alternative bei 28' angedeutet.

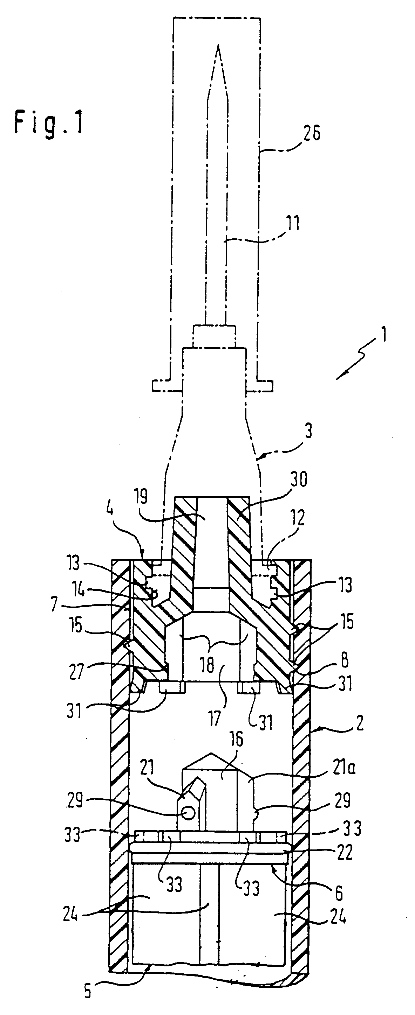

Vor einem Gebrauch wird in diesem Beispiel der Nadelhalter 4 mit der Nadelhaltehülse 3 gegensinnig zur Schraubverbindung zwischen dem Nadelhalter 4 und dem Zylinder 2 verschraubt. Dabei treten die Finger 31 in die Ausnehmungen 33 ein, laufen über die Rampen 32 hinweg, bis der in Fig. 8 dargestellte Zustand erreicht ist. Anschließend wird eine in Fig. 1 strichpunktiert angedeutete Nadelkappe 26 abgenommen.

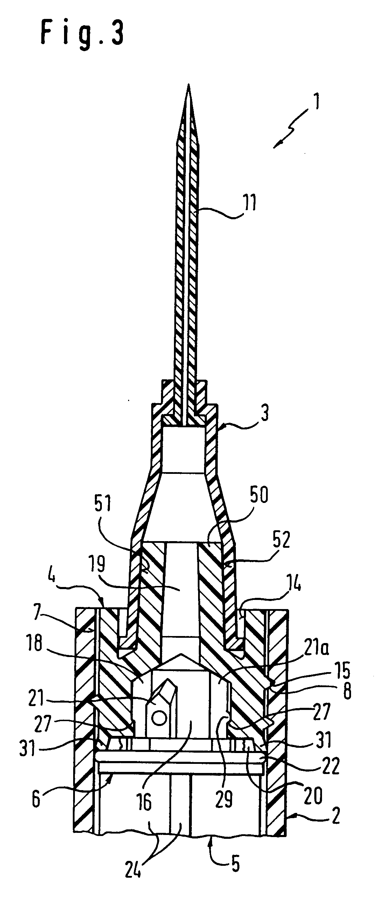

Fig. 3 zeigt ein zweites Ausführungsbeispiel, bei dem im Unterschied zum ersten Ausführungsbeispiel kein Innengewindeabschnitt 13 in einer Innenwandfläche 14 des Nadelhalters 4 vorgesehen ist. Vielmehr ist ein Ansatz 50 mit äußeren Abflachungen 51 an den Nadelhalter angeformt, auf den die entsprechende Abflachungen 52 aufweisende Nadelhaltehülse 3 dicht und unverdrehbar aufzustecken ist.

Im folgenden wird die Handhabung und Funktionsweise der Injektionsspritze 1 nach dem ersten Ausführungsbeispiel beschrieben.

Zur Schraubverbindung des Nadelhalters 4 mit dem Zylinder 2 steckt man den Nadelhalter 4 in das hintere Ende des Zylinders 2, drückt ihn dann nach vorne und dreht ihn dann etwa eine viertel bis eine volle Umdrehung im Uhrzeigersinn nach rechts, bis der Linksgewindeabschnitt 8 an der Innenwand 7 des Zylinders 2 den Linksgewindeabschnitt 15 am Außenumfang des Nadelhalters 4 fest umgriffen hat. Dann schraubt man den Flansch 12 an der Nadelhaltehülse 3 in den Linksgewindeabschnitt 13 des Nadelhalters 4. Die Schraubverbindungsrichtung zwischen der Injektionsnadel 11 und dem Nadelhalter 4 ist dabei also entgegengesetzt zur Schraubverbindungsrichtung zwischen dem Zylinder 2 und dem Nadelhalter 4.

Nachdem die Nadelhaltehülse 3 eingesetzt ist und nachdem die Nadelkappe 26, die die Injektionsnadel 11 entsprechend der strichpunktierten Linie in Fig. 1 bedeckt, entfernt ist, wird die Injektionsnadel 11 z.B. durch eine Kunststoffkappe und dann nach einem Nadelaustausch in einen Körper eingestochen, und dann wird der Kolben 6 mittels der Kolbenstange 5 nach vorne geschoben.

Nachdem die Injektion durchgeführt worden ist, wird die Injektionsnadel 11 aus dem Körper herausgezogen und mittels des Betätigungsgriffs 23 die Kolbenstange 5 nach vorne geschoben, bis die stegförmigen Drehsicherungsglieder 21 an dem vorderen Ansatz 16 des Kolbens 6 kämmend zwischen die stegförmigen Drehsicherungsglieder 18 am Nadelhalter 4 greifen und die Noppen 29 hinter die Innenschultern 27 gedrückt sind. Der Kolben 6 ist dann fest mit dem Nadelhalter 4 verbunden.

Wenn dann der Kolben 6 gegen den Uhrzeigersinn gedreht wird, gelangen die stegförmigen Drehsicherungsglieder 18 in der Höhlung 17 des Nadelhalters 4 in Kontakt mit den stegförmigen Drehsicherungsgliedern 21 am Ansatz 16 des Kolbens 6. Durch weiteres Drehen des Kolbens 6 um etwa eine viertel bis eine volle Umdrehung wird die Schraubverbindung zwischen dem Linksgewindeabschnitt 8 im Zylinder 2 und dem Linksgewindeabschnitt 15 am Nadelhalter 4 gegen die Verrastung der Finger 31 hinter den Rampen 32 gelöst.

Dann wird der Kolben 6 nach hinten gezogen, bis der nasenförmige Verriegelungsvorsprung 9 an der Innenwand 7 des Zylinders 2 in die nasenförmigen Verriegelungsvertiefungen 25 in den kreuzförmig angeordneten Führungsrippen 24 einrastet, und abgebrochen. Ein Verschieben der Nadelhaltehülse 3, des Nadelhalters 4 und des Kolbens 6 ist dann nicht mehr möglich. Die Injektionsnadel 11 liegt dabei vollständig innerhalb des Zylinders 2, so daß eine Infektion durch sie ausgeschlossen wird.

Handhabung und Funktionsweise der Injektionsnadel 11 nach dem zweiten Ausführungsbeispiel sind bis auf die Steckverbindung zwischen der Nadelhaltehülse 3 und dem vorspringenden Ansatz 30 am Nadelhalter 4 analog.

## Patentansprüche

1. Injektionsspritze (1) mit einem Zylinder (2), an dessen einem Ende ein eine Injektionsnadel (11) halternder Nadelhalter (4) anbringbar ist, mit einem in dem Zylinder (2) verschiebbaren Kolben (6), an dem eine aus dem anderen Ende des Zylinders (2) herausragende, an ihrem Ende mit einem Betätigungsgriff (23) versehene Kolbenstange (5) angebracht ist, und mit einer Vorrichtung zur selbsttätigen Verriegelung des über ein vorgegebenes Maß in Richtung zur Injektionsnadel (11) vorgeschobenen Kolbens (6) an dem Nadelhalter (4) gegenüber Zurückziehen des Kolbens (6), bei der der Nadelhalter (4) durch eine durch Hineindrehen und gegebenenfalls axiales Verschieben des Nadelhalters (4) in den Zylinder (2) zu lösende Drehverbindung (8, 15) mit dem Zylinder (2) verbindbar ist und bei der sich an dem Nadelhalter (4) und dem Kolben (6) Drehsicherungsglieder (18, 21) befinden, die bei Vorschieben des Kolbens (6) in Richtung zum Nadelhalter (4) zur Verrigelung des Kolbens (6) an dem Nadelhalter (4) ineinandergreifen und durch Verdrehen des Betätigungsglieds (23) eine Lösung der Drehverbindung (8, 15) und daraufhin ein Hineinziehen des Kolbens (6), des Nadelhalters (4) und der Injektionsnadel (11) über ihre ganze Länge in den Zylinder (2) gestatten, wobei die Drehsicherungsglieder (18, 21) Winkelabstände zueinander aufweisende, durch axiales Vorschieben des Kolbens (6) in gegenseitigen, kämmenden Eingriff zu bringende Stege (18, 21) sind,
**dadurch gekennzeichnet**,
daß zwischen dem Innenumfang des Zylinders (2) und dem Außenumfang des Nadelhalters (4) Verrastungsmittel (31, 32) vorgesehen sind, die ein verhältnismäßig leichtes Eindrehen der Drehverbindung (8, 15) zwischen dem Nadelhalter (4) und dem Zylinder (2) und ein verhältnismäßig schweres Ausdrehen der Drehverbindung (8, 15) bewirkt.

2. Injektionsspritze nach Anspruch 1, **dadurch gekennzeichnet**, daß die Verrastungsmittel (31, 32) durch unsymmetrische Nocken (32) und über diese unsymmetrischen Nocken (32) elastisch nachgiebig bewegbare Nockenfolger (31) gebildet sind.

3. Injektionsspritze nach Anspruch 2, **dadurch gekenn**zeichnet, daß die Nocken (32) die Form von Rampen (32) und die Nockenfolger (31) die Form von nachgiebigen Fingern (31) haben.

4. Injektionsspritze nach einem der vorstehenden Ansprüche, **dadurch gekennzeichnet**, daß sich die Nocken (32) am Zylinder (2) und die Nockenfolger (31) am Nadelhalter (4) befinden.

5. Injektionsspritze nach einem der Ansprüche 2 bis 4, **dadurch gekennzeichnet**, daß sich die Nocken (32) an der Innenfläche des Zylinders (2) und die Nockenfolger (31) an der zum Kolben (6) weisenden Stirnfläche des Nadelhalters (4) befinden.

6. Injektionsspritze nach Anspruch 5, **dadurch gekennzeichnet**, daß sich im Umfang eines Sockels auf dem Kolben (6) Ausnehmungen (33) zur Aufnahme der Nockenfolger (31) befinden.

7. Injektionsspritze nach dem Oberbegriff des Anspruchs 1 und insbesondere dem Kennzeichen eines der vorstehenden Ansprüche, **dadurch gekennzeichnet**, daß die Stege (21) am Kolben (6) in Richtung zum Nadelhalter (4) durch in Umfangsrichtung aufeinander zu strebende Flanken (34) zugespitzt sind.

8. Injektionsspritze nach dem Oberbegriff des Anspruchs 1 und insbesondere dem Kennzeichen eines der vorstehenden Ansprüche, **dadurch gekennzeichnet**, daß zwischen den radial nach außen gerichteten Begrenzungsflächen (20) der Stege (22) und/oder der Zwischenräume zwischen den Stegen (22) am Kolben (6) und den ihnen im Verriegelungszustand gegenüberstehenden, radial nach innen gerichteten Begrenzungsflächen der Zwischenräume zwischen den Stegen (18) und/oder der Stege (18) am Nadelhalter (4) voneinander lösbare Rastglieder (27, 29) vorgesehen sind.
